# EUROPEAN PATENT SPECIFICATION

(11) **EP 2 189 134 B1**
(45) Date of publication and mention of the grant of the patent: **22.02.2012**
(21) Application number: 09176408.4
(22) Date of filing: 18.11.2009
(51) Int. Cl.: A61F 2/16, A61B 3/10, G09B 23/28

(54) **Aspheric intraocular lens with improved control of aberrations**
Asphärische intraokulare Linse mit verbesserter Linsenfehlersteuerung
Lentille intraoculaire asphérique dotée d'un contrôle amélioré des abérrations

(30) Priority: 19.11.2008 US 116180 P
(43) Date of publication of application: 26.05.2010
(73) Proprietor: Alcon Research, Ltd., Fort Worth, TX 76134-2099 (US)
(72) Inventor: Simpson, Michael, Arlington, TX 76012 (US)
(74) Representative: Hanna, Peter William Derek

(56) References cited:
- EP-A2- 1 683 474
- WO-A2-2006/053216
- US-A1- 2006 030 938
- US-A1- 2006 227 286

## Description

### Summary

The present invention provides a method of manufacturing an intraocular lens (IOL) of a selected power, in accordance with claims which follow. A method of manufacturing an intraocular lens (IOL) of a selected power includes determining a power-specific axial separation parameter for the selected power, selecting at least one aberration correction for the IOL, designing the IOL based on the power-specific axial separation parameter to produce the selected aberration correction, and manufacturing the IOL.

In particular embodiments of the present invention, a method of designing an intraocular lens (IOL) of a selected power includes determining a power-specific axial separation parameter for the selected power. The method also includes selecting at least one aberration correction for the IOL. The method further includes designing the IOL based on the power-specific axial separation parameter to produce the selected aberration correction.

The prior art is represented by US-2006/030938 A1, WO-2006/053216 A2, EP-1,683,474 A2, and US-2006/227286 A1.

Further understanding of various aspects of the invention can be obtained by reference to the following detailed description in conjunction with the drawings, which are discussed briefly below.

### Brief Description of the Drawings

FIGURE 1 schematically depicts a model of an intraocular lens (IOL) within an eye according to a particular embodiment of the present invention;
FIGURE 2 is a table tabulating an average anterior chamber depth (ACD) for groups of patients, in which each group of patients had a particular power of IOL implanted; and
FIGURE 3 is a flow chart illustrating a method for designing an IOL according to a particular embodiment of the present invention.

### Detailed Description

Particular embodiments of the present invention provide an intraocular lens (IOL) designed to correct aberration using a calculated anterior chamber depth that varies with IOL power. Such embodiments may advantageously improve the image contrast of the IOL resulting in improved vision for the patient. The term "intraocular lens" and its abbreviation "IOL" are used herein interchangeably to describe lenses that are implanted into the interior of the eye to either replace the eye's natural lens or to otherwise augment vision regardless of whether or not the natural lens is removed. Intracorneal lenses and phakic intraocular lenses are examples of lenses that may be implanted into the eye without removal of the natural lens.

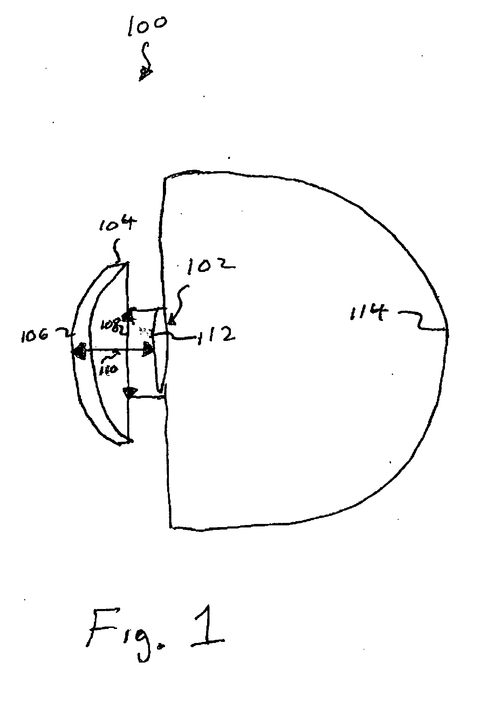

FIGURE 1 schematically illustrates a model 100 of an IOL 102 within an eye. The model 100 includes a cornea 104 having an anterior surface 106. A pupil diameter 108 allowing light to travel to the IOL 102 may also be included in the model 100. The relative position of the IOL 102 and the cornea 104 may be quantified by an anterior chamber depth (ACD) 110 describing an axial distance between the anterior surface 106 of the cornea 104 and an anterior surface 112 of the IOL 102. Although the following description will be explained in terms of ACD, it should be understood that any parameter corresponding to a relative axial position between the cornea 104, IOL 102, and retina 114 (hereinafter referred to as an "axial separation parameter"), including ACD, may be used in any of the embodiments of the present invention described herein. In particular, the axial separation parameter may be back-calculated from measurements of eye length, corneal power, corneal asphericity, implanted IOL power, and postoperative refractive error to determine relative position for purposes of the model 100. In the model 100, the ACD 110 is selected to approximate the relative axial position of the IOL 102 when actually implanted in a living eye. The model 100 is used to determine how light rays will converge on the retina 114 to produce visible images.

Previous techniques for modeling IOLs have determined the ACD based on physical approximations of where the IOL would be disposed in a typical patient. An IOL is held in a particular position in the eye by haptics that contact particular anatomical features in the eye. For example, in the case of a foldable IOL used to replace the natural lens of an eye (also known as an "aphakic IOL"), the IOL can be held in place within the capsular bag. Similarly, in the case of an IOL that works in conjunction with the natural lens (also known as a "phakic IOL"), the haptics might contact the angle of the eye to hold the IOL in place. Because there may be variations in the relative position of these anatomical features of the eye relative to the cornea, the actual ACD of the IOL as implanted in the eye can vary from patient to patient. In designing IOLs, previous techniques attempted to develop a "best fit" for the ACD by determining an average over a large number of patients of the relative position of the cornea to these anatomical features that hold the IOL. A nominal value for the ACD based on such anatomical variations would typically be around 4.5 mm for an ordinary patient population.

One difficulty faced by IOLs designed according to such a methodology is that IOLs are often designed to control aberration through the use of aspheric surfaces, toric surfaces, diffractive structures, and the like. Such controlled aberration is particularly desirable to produce a desired depth of field for vision and to reduce or eliminate visible phenomena such as blurring, glares, halos, and the like. However, aberration corrections are quite sensitive to the axial position of the IOL as represented by the ACD. In real patients, the actual axial position of the IOL can range from as low as 3.5 mm to as high as 7.0 mm. These anatomical variations between patients can cause significant variation in the convergence of light at the retina produced by the IOL, which can cause significant variation in lens performance from patient to patient.

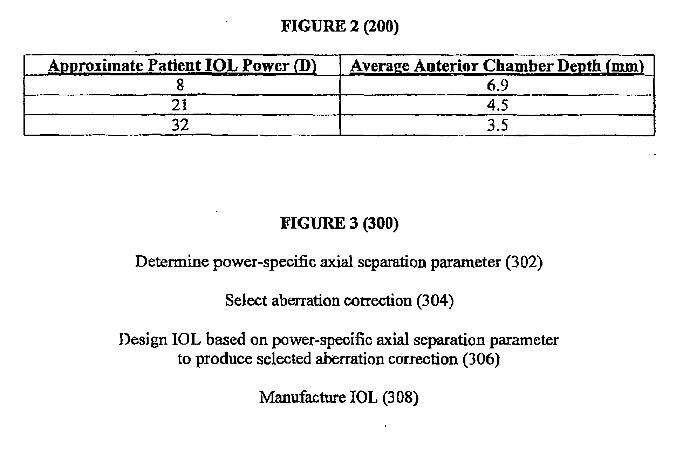

Techniques for designing an IOL according to particular embodiments of the present invention employ a modified ACD that also takes into account the power of the IOL. These techniques exploit a phenomenon that had not previously been remarked in IOL design methodologies, which is that patients requiring a higher power IOL frequently have a lower ACD than those requiring lower power IOLs. The table 200 in FIGURE 2, which illustrates this phenomenon, tabulates results for a particular study of patient groups using different powers of ACRYSOF® IOLs, showing an average ACD for each patient group. The groups are collections of patients with similar implanted IOL powers, with the nominal IOL power for the group being an average of the various IOL powers within that group. Consequently, higher power IOL lenses can be designed for aberration control based on a lower (and therefore more accurate) ACD value determined for the particular lens power. For example, an IOL with a power of at least 25 D could have an ACD value less than 4 mm. Similarly, an IOL with a power less than 15 D could have an ACD value greater than 5.5 mm.

FIGURE 3 is a flow chart 300 showing a method for designing and/or manufacturing an IOL according to a particular embodiment of the present invention. At step 302, a power-specific axial separation parameter is determined for an IOL. The power-specific axial separation parameter may be, for example, an average ACD value for a patient population in which IOLs of that power have been implanted. It may also be calculated for patient populations with similar IOL powers that also have similar axial lengths or corneal powers to each other. In another example, the power-specific value may be interpolated from average ACD values for different IOL powers. In general, the determination of a power-specific value involves any measurement of an actual axial separation parameter that is specifically associated with an IOL having a specific power and that is subsequently used to calculate a theoretical ACD based on IOL power. At step 304, a desired aberration correction is selected. This may be determined, for example, by specifying the desired image contrast, at the macula of the retina, or by specifying other parameters such as the level of spherical aberration, the correction or control of astigmatism, the creation of a desired depth of field, or to produce any other desired modification of aberration. Furthermore, the desired aberration correction may include multiple different types of aberration correction in combination. At step 306, the IOL is designed based on the power-specific axial separation parameter to produce the selected aberration correction. The IaL may then be manufactured at step 308.

While the foregoing discussion has specifically addressed using the connection between an axial separation parameter and the power of an implanted IOL to determine a power-specific axial separation parameter, it should also be noted that the connection between the axial separation parameter and IOL power can also be exploited in other ways. In particular, the relationship among optical properties of the cornea (e.g., asphericity, power), eye length, and IOL power may be used to determine both the power and the axial separation parameter used in the eye model. Thus, it is possible to have a method for selecting an IOL that includes determining eye length and at least one optical property of a cornea, determining an IOL power and an aberration correction based on the eye length and the at least one optical property of the cornea, and implanting an IOL having the selected power and the aberration correction. Similarly, this concept could extend to a method for designing and/or manufacturing an IOL that includes selecting an eye length and at least one optical property of a cornea, determining an IOL power and an aberration correction based on the eye length and the at least one optical property of the cornea, and designing and/or manufacturing an IOL with the IOL power and the aberration correction. Such a method may include grouping patients by a combination of eye length and corneal properties so that, for example, an eye of shorter length but higher corneal power might use a lens with a power normally associated with a lesser corneal power III an average eye.

The present invention has been described by reference to certain preferred embodiments; however, it should be understood that it may be embodied in other specific forms or variations thereof without departing from its essential characteristics.

## Claims

1. A method of manufacturing intraocular lenses (IOLs) (102) of different selected powers, comprising:
determining (302) a power-specific axial separation parameter for each of the selected powers;
selecting (304) at least one aberration correction for a particular IOL;
designing (306) each of the IOLs based on the values of the power-specific axial separation parameter to produce the selected aberration correction for the respective power-specific axial separation parameter; and
manufacturing (308) the IOLs in accordance with the designs.

2. The method of Claim 1, wherein the power-specific axial separation parameter is an anterior chamber depth (ACD).

3. The method of claim 2, wherein the power-specific axial separation parameter is based on an average ACD value for a patient population in which IOLs of that power have been implanted.

4. The method of Claim 2, wherein the selected power is at least 25 D and the anterior chamber depth is less than 4.0 mm.

5. The method of Claim 2, wherein the selected power is less than 15 D and the anterior chamber depth is at least 5.5 mm.

6. The method of Claim 1, wherein the particular IOL is an aphakic IOL.

7. The method of Claim 1, wherein the particular IOL is a phakic IOL.

8. The method of Claim 1, wherein the aberration correction for a particular IOL is produced by an aspheric surface of the IOL.

9. The method of Claim 1, wherein the aberration correction for a particular IOL is produced by a toric surface of the IOL.

10. The method of Claim 1, wherein the aberration correction for a particular IOL is determined based on a selected depth of field for the IOL.

11. The method of any one of claims 1 to 10, comprising:
calculating the axial separation parameter from measurements of eye length, corneal power, corneal asphericity, implanted IOL power, and postoperative refractive error for purposes of determining the relative position of the IOL (102) and the cornea (104) in a model eye (100);
quantifying an anterior chamber depth (ACD) (110) describing an axial distance between the anterior surface (106) of the cornea (104) and an anterior surface (112) of the IOL (102) in the model eye (100); and
selecting an ACD (110) to approximate the relative axial position of the IOL (102) when actually implanted in a living eye.

12. The method of any one of claims 1 to 10, further comprising:
determining an eye length and at least one optical property of a cornea (104) for a given patient, so as to calculate the axial separation parameter;
determining an IOL power and an aberration correction based on the eye length and the at least one optical property of the cornea.

## Patentansprüche

1. Verfahren zum Herstellen intraokularer Linsen (IOLs) (102) mit unterschiedlichen ausgewählten Brechkräften, mit den Schritten:
Ermitteln (302) eines brechkraftspezifischen axialen Abstandsparameters für jede von den ausgewählten Brechkräften;
Auswählen (304) wenigstens einer Abberations-Korrektur für eine spezielle IOL;
Entwerfen (306) jeder von den IOLs auf der Basis der Werte des brechkraftspezifischen axialen Abstandsparameters, um die ausgewählte Abberations-Korrektur für den entsprechenden brechkraftspezifischen axialen Abstandsparameter zu erzeugen; und
Herstellen (308) der IOLs gemäß den Entwürfen.

2. Verfahren nach Anspruch 1, wobei der brechkraftspezifische axiale Abstandsparameter eine Tiefe der vorderen Augenkammer (ACD) ist.

3. Verfahren nach Anspruch 2, wobei der brechkraftspezifische axiale Abstandsparameter auf einem durchschnittlichen ACD-Wert für eine Patientenpopulation basiert, in welchen IOLs mit dieser Brechkraft implantiert worden sind.

4. Verfahren nach Anspruch 2, wobei die ausgewählte Brechkraft wenigstens 25 D ist und die Tiefe der vorderen Augenkammer kleiner als 4,0 mm ist.

5. Verfahren nach Anspruch 2, wobei die ausgewählte Brechkraft kleiner als 15 D ist und die Tiefe der vorderen Augenkammer weinigstens 5,5 mm ist.

6. Verfahren nach Anspruch 1, wobei die spezielle IOL eine aphakische IOL ist.

7. Verfahren nach Anspruch 1, wobei die spezielle IOL eine phakische IOL ist.

8. Verfahren nach Anspruch 1, wobei die Abberations-Korrektur für eine spezielle IOL durch eine asphärische Oberfläche der IOL erzeugt wird.

9. Verfahren nach Anspruch 1, wobei die Abberations-Korrektur für eine spezielle IOL durch eine torische Oberfläche der IOL erzeugt wird.

10. Verfahren nach Anspruch 1, wobei die Abberations-Korrektur für eine spezielle IOL auf der Basis einer ausgewählten Schärfentiefe für die IOL ermittelt wird.

11. Verfahren nach einem der Ansprüche 1 bis 10, mit den Schritten:
Berechnen des axialen Abstandsparameters aus Messungen der Augenlänge, der Hornhautbrechkraft, der Hornhautasphärizität, der Brechkraft der implantierten IOL und dem postoperativen Brechungsfehler für Zwecke der Ermittlung der relativen Position der IOL (102) und der Hornhaut (104) in einem Modellauge (100);
Quantifizieren einer Tiefe der vorderen Augenkammer (ACD) (110), die einen axialen Abstand zwischen der vorderen Oberfläche (106) der Hornhaut (104) und einer vorderen Oberfläche (112) der IOL (102) in dem Modellauge (100) beschreibt; und
Wählen einer ACD (110), um die relative axiale Position der IOL (102) anzunähern, wenn sie tatsächlich in ein lebendes Auge implantiert wird.

12. Verfahren nach einem der Ansprüche 1 bis 10, ferner mit den Schritten:
Ermitteln einer Augenlänge und wenigstens einer optischen Eigenschaft einer Hornhaut (104) für einen gegebenen Patienten, um so den axialen Abstandsparameter zu berechnen;
Ermitteln einer IOL-Brechkraft und einer Abberations-Korrektur auf der Basis der Augenlänge und der wenigstens einen optischen Eigenschaft der Hornhaut.

## Revendications

1. Procédé de fabrication de lentilles intraoculaires (IOL) (102) de différentes puissances sélectionnées, comprenant les étapes consistant à :
déterminer (302) un paramètre de séparation axiale spécifique à la puissance pour chacune des puissances sélectionnées ;
sélectionner (304) au moins une correction d'aberration pour une lentille IOL particulière ;
concevoir (306) chacune des lentilles IOL sur la base du paramètre de séparation axiale spécifique à la puissance pour produire la correction d'aberration sélectionnée pour le paramètre de séparation axiale spécifique à la puissance respectif ; et
fabriquer (308) les lentilles IOL conformément aux conceptions.

2. Procédé selon la revendication 1, dans lequel le paramètre de séparation axiale spécifique à la puissance est une profondeur de chambre antérieure (ACD).

3. Procédé selon la revendication 2, dans lequel le paramètre de séparation axiale spécifique à la puissance est basé sur une valeur d'ACD moyenne pour une population de patients dans laquelle des lentilles IOL de cette puissance ont été implantées.

4. Procédé selon la revendication 2, dans lequel la puissance sélectionnée est au moins 25 D et la profondeur de chambre antérieure est inférieure à 4,0 mm.

5. Procédé selon la revendication 2, dans lequel la puissance sélectionnée est inférieure à 15 D et la profondeur de chambre antérieure est au moins de 5,5 mm.

6. Procédé selon la revendication 1, dans lequel la lentille IOL particulière est une lentille IOL aphaque.

7. Procédé selon la revendication 1, dans lequel la lentille IOL particulière est une lentille IOL phaque.

8. Procédé selon la revendication 1, dans lequel la correction d'aberration pour une lentille IOL particulière est produite par une surface asphérique de la lentille IOL.

9. Procédé selon la revendication 1, dans lequel la correction d'aberration pour une lentille IOL particulière est produite par une surface torique de la lentille IOL.

10. Procédé selon la revendication 1, dans lequel la correction d'aberration pour une lentille IOL particulière est déterminée sur la base d'une profondeur de champ sélectionnée pour la lentille IOL.

11. Procédé selon l'une quelconque des revendications 1 à 10, comprenant les étapes consistant à :
calculer le paramètre de séparation axiale à partir des mesures de longueur de l'oeil, de puissance de la cornée, d'asphéricité de la cornée, de la puissance de la lentille IOL implantée et de l'erreur de réfraction postopératoire dans le but de déterminer la position relative de la lentille IOL (102) et de la cornée (104) dans un oeil modèle (100);
quantifier une profondeur de chambre antérieure (ACD) (110) décrivant une distance axiale entre la surface antérieure (106) de la cornée (104) et la surface antérieure (112) de la lentille IOL (102) dans l'oeil modèle (100) ; et
sélectionner une ACD (110) pour approximer la position axiale relative de la lentille IOL (102) lorsque qu'elle est réellement implantée dans un véritable oeil.

12. Procédé selon l'une quelconque des revendications 1 à 10, comprenant en outre les étapes consistant à :
déterminer une longueur d'oeil et au moins une propriété optique d'une cornée (104) pour un patient donné, de manière à calculer le paramètre de séparation axiale ;
déterminer une puissance de lentille IOL et une correction d'aberration sur la base de la longueur de l'oeil et d'au moins une propriété optique de la cornée.
